Europäisches Patentamt

(19) European Patent Office

Office européen des brevets

(11) Publication number: **0 063 661**
**B1**

# EUROPEAN PATENT SPECIFICATION

(45) Date of publication of patent specification: 21.11.85

(21) Application number: **81305583.7**

(22) Date of filing: **25.11.81**

(51) Int. Cl.⁴: $A\ 61\ K\ 39/135$

(54) Process for inactivating foot-and-mouth disease virus (FMDV).

(30) Priority: **25.11.80 AR 283355**

(43) Date of publication of application:
**03.11.82 Bulletin 82/44**

(45) Publication of the grant of the patent:
**21.11.85 Bulletin 85/47**

(84) Designated Contracting States:
**BE CH DE FR GB LI NL**

(56) References cited:
**GB-A- 894 623**

**INDUSTRIAL MICROBIOLOGY ABSTRACTS,
vol. 1, 1965, page 58, ref. 1747; O.N.
FELLOWES: "Foot and mouth disease virus".
Applied Microbiology 14 (2) 1966, pag. 206-211**

(73) Proprietor: **Latorre, Jose Leonardo**
**Serrano 661**
**Buenos Aires (AR)**
(73) Proprietor: **Denoya, Claudio**
**Serrano 661**
**Buenos Aires (AR)**
(73) Proprietor: **Scodeller, Eduardo**
**Serrano 661**
**Buenos Aires (AR)**
(73) Proprietor: **Vasquez, Cesar**
**Serrano 661**
**Buenos Aires (AR)**
(73) Proprietor: **Lebendiker, Mario**
**Serrano 661**
**Buenos Aires (AR)**
(73) Proprietor: **Dubra, Maria Susana**
**Serrano 661**
**Buenos Aires (AR)**
(73) Proprietor: **Crespo, Oscar**
**Serrano 661**
**Buenos Aires (AR)**
(73) Proprietor: **CONSEJO NATIONAL DE
INVESTIGACIONES CIENTIFICAS Y TECNICAS**
**Rivadavia 1917**
**Buenos Aires (AR)**
(73) Proprietor: **FUNDACION PARA LA EDUCACION,
LA CIENCIA Y LA CULTURA**
**Moreno 431**
**Buenos Aires (AR)**

Courier Press, Leamington Spa, England.

(72) Inventor: **Latorre, Jose Leonardo**
**Serrano 661**
**Buenos Aires (AR)**
Inventor: **Denoya, Claudio**
**Serrano 661**
**Buenos Aires (AR)**
Inventor: **Scodeller, Eduardo**
**Serrano 661**
**Buenos Aires (AR)**
Inventor: **Vasquez, Cesar**
**Serrano 661**
**Buenos Aires (AR)**
Inventor: **Lebendiker, Mario**
**Serrano 661**
**Buenos Aires (AR)**
Inventor: **Dubra, Maria Susana**
**Serrano 661**
**Buenos Aires (AR)**
Inventor: **Crespo, Oscar**
**Serrano 661**
**Buenos Aires (AR)**

(74) Representative: **Ablewhite, Alan James et al**
**MARKS & CLERK 57/60 Lincoln's Inn Fields**
**London WC2A 3LS (GB)**

## Description

The present invention relates to an new process for inactivating foot-and-mouth disease virus, (FMDV), based on the specific activation of an endonuclease contained in said viruses, which is capable of degrading the RNA responsible for viral replication. When, by degradation of the genomic RNA, the virus is rendered incapable of replication, it is quickly inactivated and is therefore non-infective, thus becoming a suitable material for preparing a foot-and-mouth disease vaccine substantially free from residual infective or toxic effects and possessing a high antigenic activity.

The process of the present invention affords the possibility of replacing traditional methods of inactivation, based on the use of formaldehyde, AFI (acetylethyleneimine) or BEI (binary ethyleneimine) as inactivant agents, by means of the application of other agents which do not alter the capacity of the proteins in the viral capsid or the structure of the virus.

### Introduction

The foot-and-mouth disease virus, a member of the picornavirus family, is composed by a single-stranded RNA surrounded by a protein capsid. This capsid contains 60 copies of each of the main structural polypeptides $VP_1$, $VP_2$, $VP_3$, and $VP_4$.

During the process of inactivation to degrade the genomic RNA and inhibit the infectivity of the virus there occur alterations of the protein structure of the capsid which affect the immunogenicity of the vaccine. Since 1926, formaldehyde has been used as an inactivant for the virus (1) (for references see below) but it is known that formalinized vaccines contain residual infective particles (2, 3). AEI (acetylethyleneimine) and BEI (binary ethyleneimine) (4, 5) are better inactivants, since the infectivity of the virus is destroyed by a first order reaction. However, some strains of the virus have proved unstable when treated with these agents, with marked deterioration of antigenicity after the inactivation (6). Also both inactivants are highly toxic.

Research carried out at the Centro de Virologia Animal (CEVAN) led to the detection in purified foot-and-mouth disease virions of the existence of an endoribonuclease. This was the first description of the existence of a potentially active enzyme located with a picornavirus (8, 9). This viral endoribonuclease can be activated to fragment specifically the genome, leaving fully intact capsids with immunogenic capacity, suitable for their use as vaccines.

1. Vallee, H., Carre, H., and Rinjard, J. (1926). Sur l'immunisation antiaphteuse par le virus formale. *Rev. Gen. Med Vet. 35,* 128.
2. Moosbrugger, G. A. (1948). Recherches experimentales sur la fievre aphteuse. *Schweiz. Arch. Tierheilk. 90,* 176.
3. Schneider, B. (1955). Zur Infektiositet der Maul-und-Klausen der Absorbant Vakzine. *Mn. Tierheilk. 7,* 81.
4. Brown, F., Hyslop, N. Sr. G., Crick, J. and Morrow, A. W. (1963). The use of Acetyl-ethylene in the production of inactivated Foot-and-Mouth disease vaccines. *J. Hyg. Camb.* 61, 337.
5. Bahnemann, H. G. (1975). Binary ethyleneimine as an inactivant for Foot-and-Mouth disease virus and its application for vaccine production. *Arch. Virol. 47,* 47.
6. Staple, R. F., Morrow, A. W., and Fletton, B. W. (1975). The effect of acetyleneimine upon a strain of inactivated FMDV virus stored at 4°. *Arch. Virol.* 47, 331.
7. Girard, H. C. Byramoglu, O., Erol, N. and Burgut, H. (1977). Inactivation of O, FMDV virus by the binary Ethyleneimine (BEI). *Bull of Int. Epiz.* 87, 201.
8. Denoya, C. D., Scodeller, E. A., Gimenez, B. H., Vazquez, C. and La Torre, J. L. (1978). Foot-and-mouth disease virus. 1) Stability of its ribonucleic acid. *Virology 84* 230.
9. Denoya, C. D., Scoddeler, E. A. Vasquez, C. and L. A. Torre, J. L. Foot-and-mouth disease virus. II) Endoribonuclease activity within purified virions. *Virology 57,* 153.

The influence of salts on foot and mouth disease virus was discussed by O. N. Fellowes (*Applied Microbiology,* 14: 206—211, 1966). This paper was concerned with the stability of stored virus lines. It considered the effect on stability of $Na^+$ and $Mg^{2+}$ ions. 1 and 2M concentrations of Mg markedly slowed the degradation of bovine-passaged virus, but 1 and 2M concentrations of Na showed a slight retardation of degradation.

It has now been found that whereas Na has little if any effect on inactivating tvucvirus, especially at lower concentrations, e.g. 100mM or more, other monovalent ions appear to have a pronounced effect.

### Development of this invention

Experiments in activating the enzyme in the open virion (released by heat and/or pH) were performed and activation by monovalent cations such as ammonium, cesium and potassium at concentrations as low as 100 mM, was found. The addition of bivalent cations such as $Mg^{2+}$ and $Ca^{2+}$ (5 mM) produced greater activation of the enzyme. In the open virion, particularly good activation was obtained with the 100 mM monovalent+5 mM bivalent combination.

According to this invention there is provided a process for inactivating foot-and-mouth disease virus, characterised in that it comprises incubating said virus in the presence of ammonium, potassium or cesium cations provided by a source present at a concentration of at least 100 m molar and at the temperature of 30—40°C and then recovering inactivated stable virus. The incubation is preferably effected at a pH of about 8.5.

It was also found that non-ionic detergents such as polyethylene glycol alkylphenylethers e.g. Triton X-100 (Trade Mark for a brand of polyethylene glycol p-isooctyl phenyl ether), at concentrations of between 0.1 and 0.5%, NP40, BRIJ (Trade Mark), Tween (Trade Mark), and other similar substances are other potent activators of the enzyme, particularly in the specific case of the type C foot-and-mouth disease virus. Tests carried showed that, for example, PEG alkylphenyl ethers such as Triton® X-100 are real "*in vitro*" activators of viral nuclease. On the other hand, Triton® X-100 quickly disperses virus clusters, permitting a better interaction of these with the ammonium cation. This has been evidenced several times by electron microscopy. Similarly, the recovery of virions was substantially greater in multiple tests carried out in the laboratory.

Investigations were made as to the possibility of producing 140S particle mass with degraded RNA. Three roller bottles containing a monolayer of BHK 21 (baby hamster kidney) cells obtained from CEPANZO (Centro Panamericano de Zoonosis, Argentina) were infected at low multiplicity with $A_{24}$ Cruzeiro virus obtained from SELAB. The infection was left overnight, one roller bottle was infected in the presence of $^3$H-uridine, so as to label the virus. The cellular fluids were centrifuged for 15 min at 12,000 rpm (1000G) and the supernatant fluids were pooled and divided into 5 aliquots of 2.5 ml each. Each aliquot was treated with a medium as follows:

A  Control Eagle's medium only  0° 14 h

B  Control Eagle, Tris 100 mM,  0° 14 h
   $NH_4Cl$ 500 mM (pH 8.5)

C  Incubated Eagle, Tris 100 mM,  37° 14 h
   $NH_4Cl$ 500 mM (pH 8.5)

D  Incubated Eagle, Tris 100 mM,  37° 1 h
   $NH_4Cl$ 500 mM (pH 8.5)

E  Incubated Eagle, Tris 100 mM,  1 h
   $NH_4Cl$ 500 mM,
   Triton 0.5% (pH 8.5)

After respective treatments, each sample was purified as follows for the purpose of obtaining 140S particles:

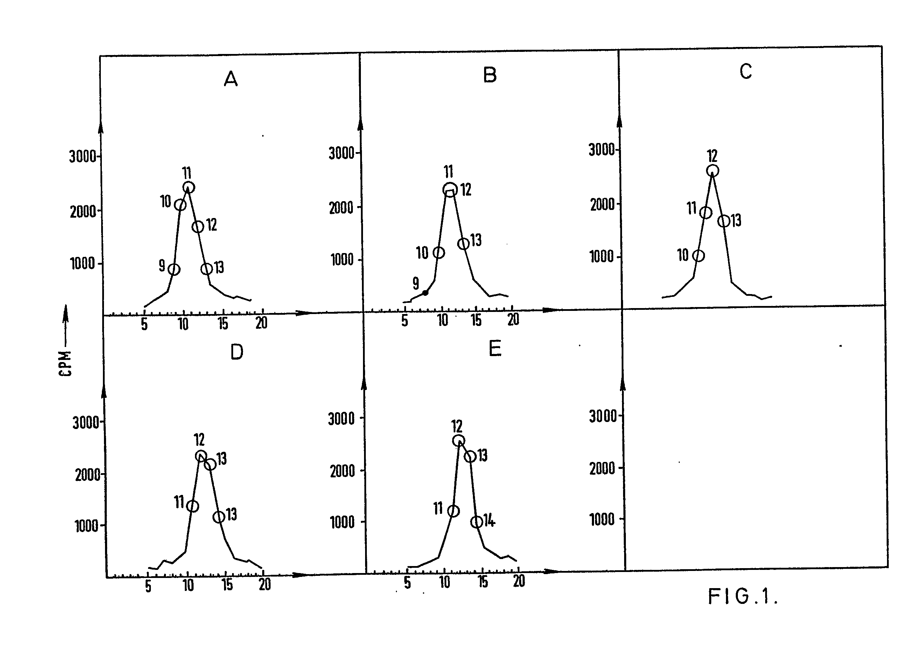

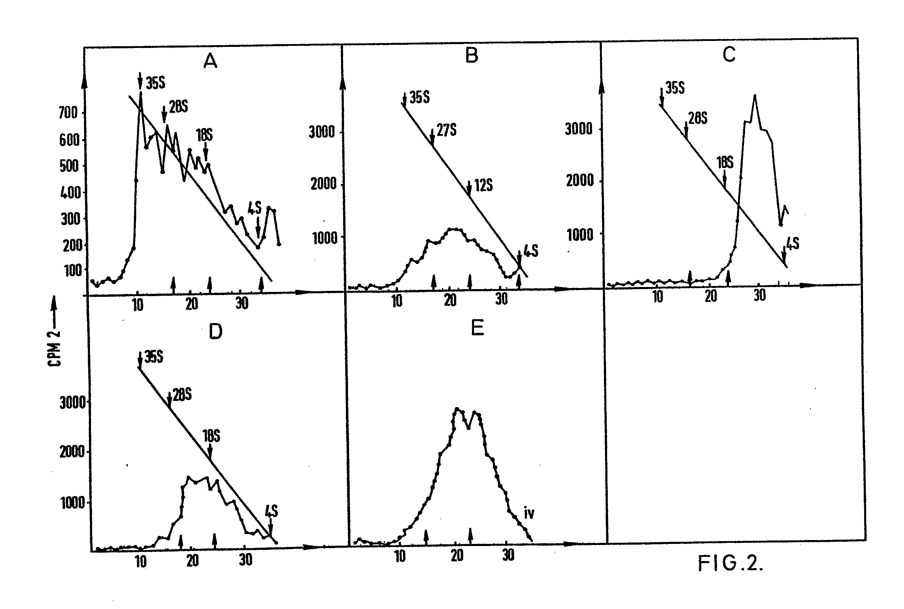

Each fraction was brought to 20 mM EDTA (neutralised to pH 7.4) and to 0.2% Sarcosyl (Trade Mark) NL 97 (Geigy), and layered onto 10—30% (w/v) linear sucrose gradient made in NET buffer (100 mM NaCl, 1 mM EDTA 50 mM Tris-HCl, pH 7.4). The samples were run in an SW27 rotor (Beckman ultracentrifuge) for 3.5 h at 26,000 rpm (150,000G) and at 2°. The total radioactivity of each aliquot of the gradient was counted in a liquid scintillation counter. The data obtained show that recovery of 140S particles was not significantly affected by the various treatments (Fig. 1) (the experiment was repeated 5 times with like results). The fractions of each gradient containing virus were pooled, brought to 1% (w/v) with SDS and extracted twice with phenol-chloroform at 60°C, the extracted RNA was precipitated with alcohol, resuspended in NET buffer+0.1% SDS and analysed in sucrose gradients made in NET-SDS, 10—30% (w/v), and run at 20°, 22,000 rpm (105000G), 14 h 30 min. Nonradio-active ribosomal RNA and 4S were used in each gradient as internal markers. Intense degradation of 35S RNA into fragments of low molecular weight was observed in the virions which had been treated with $NH_4Cl$ (Fig. 2).

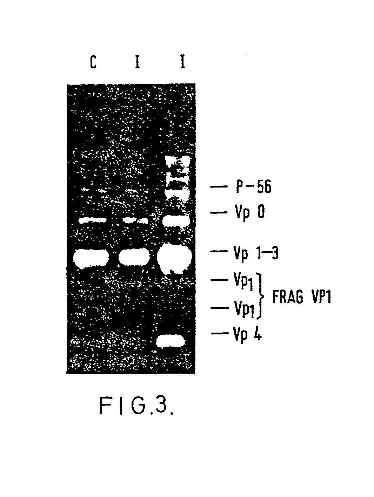

However, because of the sensitivity of the methods used, it was not possible to be certain of total absence of infective 35S RNA. It was therefore decided that the biological immunity tests described below should be undertaken. In addition, the intactness of the viral particles was examined by electron microscopy and the preservation of the immunogenic protein was investigated by analysis of the proteins in polyacrylamide gels (for which purpose the virus was labeled with a mixture of $^3$H amino acids). No differences between control and treated inactivated virus were observed (Fig. 3), demonstrating that the virion would be suitable for further processing into a vaccine.

With intact 140S particles with their RNA degraded, the safety of the virus by biological methods, and the preparation of an experimental vaccine, were investigated.

An important point was the utilisation of biological tests to adjust the inactivation time of the virus. $A_{24}$ Cruzeiro virus originally obtained at SELAB was used. This virus was then replicated on BHK monolayer and used in successive passages (approximately 30 passages in BHK monolayer). The virus was produced normally in roller bottles ($3.5 \times 10^8$ cells each) and in all cases the clarified fluid was used for centrifugation for 10 min at 10,000 rpm (9750G); the resultant supernatant fluid wysthen always used. The viruses were plated in petri dishes or on flasks, by conventional techniques.

The main subject of this invention is, therefore, process for inactivating foot-and-mouth disease virus, characterised in that it comprises incubating said virus in the presence of ammonium or Group 1A metal cations e.g. provided by soluble ammonium salts and Group 1A salts, if desired in the presence of a non-ionic detergent and $Ca^{2+}$ or $Mg^{2+}$ ions in the culture medium where the virus was produced.

Example
1) Safety testing by plating on monolayer

$A_{24}$ Cruzeiro virus from one roller bottle was divided into two aliquots of 10 ml each. One was used as control and was kept to 0° for 18 h. The other aliquot was inactivated by the addition of 100 mM tris and 500 mM of $NH_4Cl$ at pH 8.5 (final pH of the inactivation reaction) and by incubation at 37° for 18 h. Both viruses were pelleted out at 49,000 rpm (200 G) in a fixed-angle 50 Ti rotor for 60 min at 4°. The incubation was carried out also in the presence of 0.2% Triton® X-100 and $Mg^{2+}$ 2 mM. The pellets were resuspended in 1 ml (10× the original) of Eagle's medium. The viruses were finally diluted in base 10 and plated.

Titre of control virus: $1×10^7$ PFU/ml.

Titre of inactivated virus: negative at all dilutions.

Titre of virus inactivated with Triton® X-100+$Mg^{2+}$: negative at all dilutions.

### 2) Inactivation kinetics

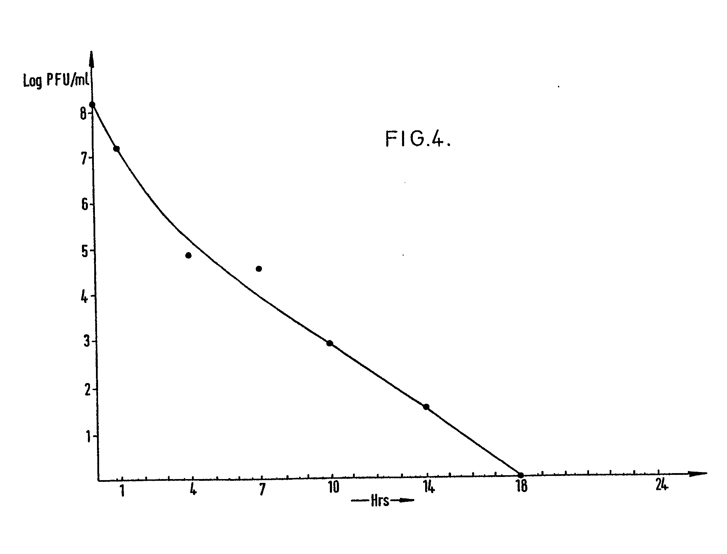

$A_{24}$ Cruzeiro virus (see above) in clarified fluids from 4 roller bottles was pooled and divided into 7 aliquots of 10 ml each. The material was stored at 0° until use. The inactivation times were 1, 4, 7, 10, 14 and 18 hours. In all cases, the same inactivation procedure as described above was used (Tris 100 mM, $NH_4Cl$ 500 mM, pH 8.5). The inactivations were performed at set intervals so that all the treatments ended simultaneously. Figure 4 shows the inactivation curve obtained. This curve was repeated three times with similar results. When the end point was extended to 20, 24, and 26 h, negative results were obtained (5 runs). In this case Triton® X-100 and $Mg^{2+}$ did not alter the results either. Comparable results were obtained when the 500 mM $NH_4Cl$ was replaced by 500 mM cesium chloride or 500 mM potassium chloride.

### 3) Safety test in the unweaned mouse

$A_{24}$ Cruzeiro virus (see above) was used in 5 to 8 day unweaned mice of Rowland breed obtained from private breeders in Argentina. The virus from one roller bottle was clarified and two aliquots of 5 ml each were separated out. One was used as control and the other was inactivated as stated above for 20 h in the presence of Triton® X-100 and Mg $Cl_2$.

The inactivated virus was raised to 5 times the concentration by pelleting. The control virus was used direct. Each mouse was injected with 0.125 ml of the virus intraperitoneally (there was no deaths from traumas). Control tests were performed with virus-free Eagle's medium.

| Results | 24 h I/S | 48 h I/S | 168 h I/S |
|---|---|---|---|
| Eagle's medium only 5 mice injected | 5/5 | 5/5 | 5/5 |
| Mother A | | | |
| Virus 1× | 5/5 | 5/0 | — |
| Eagle's medium only 5 mice injected | 5/5 | 5/5 | 5/5 |
| Mother B | | | |
| Inactivated virus (5×) | | | |
| 5 mice injected | 5/5 | 5/5 | 5/5 |

I=Injected
S=Surviving

### 4) $C_3$ Resende

It was decided that a wild strain should be studied and $C_3$ Resende virus, which was obtained passed through guinea pigs from SELAB (SENASA) was selected. The virus had undergone 6 passages through guinea pig. At the Centro de Virologia Animal it was passed through guinea pig in passages 7, 8 and 9 by conventional techniques. Virus from the 7th passage in guinea pig was adjusted to BHK monolayer in 5 passages (working stock). One aliquot was inactivated as already described for $A_{24}$, but with the addition of 0.1% of Triton® X-100 and 2 mM Mg/$Cl_2$, for 24, 26 and 28 h. The virus was pelleted and concentrated 10×, resuspended in Eagle salts medium without Triton and plated by a conventional technique.

Titre of control
24 h $2.5×10^9$ PFU/ml.
26 h $3.25×10^9$ PFU/ml.
28 h $2.75×10^9$ PFU/ml.

In all cases the dilutions of virus inactivated for 20 h were negative.

### 5) Inactivation kinetics of $C_3$ Resende virus

Inactivation was performed in Tris-ammonium-Triton® buffer at 37° for 4, 8, 18 and 24 h, with virus from a single viral infection. At the end of the activation the virus was pelleted and resuspended 10× in Eagle's medium and plated.

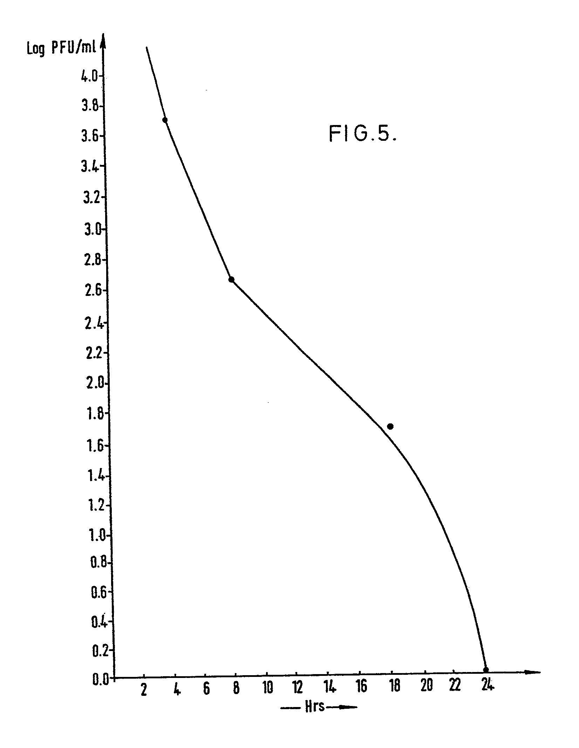

The titre of the control was upwards of $10^9$ PFU/ml (Fig. 5). In addition, a single tissue culture test was performed (monolayer Roux $1×10^8$ cells). Three serial passages were performed and were negative as judged by observation of the cells under an optical microscope.

### 6) Inactivation of the $C_3$ Resende virus in the absence of Triton® X-100

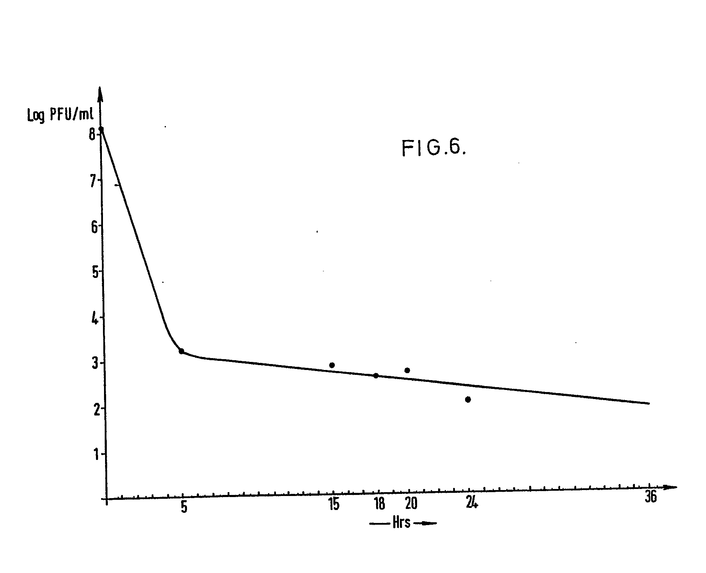

The inactivation shown in Figure 5 was performed in the presence of Triton® X-100. The basis of the utilisation of this non-ionic detergent results upon the fact that the type C viruses tested—i.e. $C_3$ Resende—are inactivated with kinetics of the second order in the absence of the detergent (Fig. 6). In view of this result, the Centro de Virologia Animal standardised its techniques using Triton® X-100 on a regular basis in all its subsequent experiments and with a view to obtaining in all cases inactivations tending to zero risks (said Fig. 5 corresponds to the inactivation kinetics ($C_3$ Resende) in Tris-ammonium-Triton®-$Mg^{2+}$-Log PFU/ml vs. residual activity per inactivation).

### 7) Effects of $Mg^{2+}$ and $Ca^{2+}$ ions

These ions reinforce the activating action of ammonium salts on viral nuclease in previously opened virions.

In intact virus this effect is less noticeable. Similar results are obtained with $Ca^{2+}$ in identical concentrations. However, the total of these ions, in concentrations of 2 to 5 mM during the incubation of intact virions in $NH_4Cl$ 500 mM,

showed under the electron microscope a better preservation of the capsid structure.

8) Preparation of an experimental vaccine

Four roller bottles were infected with $C_3$ Resende (5th passage in BHK monolayer) and a pool of clarified viral suspension was obtained as already described. The pooled material was divided into two aliquots, one as control to obtain the titre of virus and the other for inactivation. The latter aliquot was inactivated with the buffer specified above for 28 hr at 37° and was stored at 0° until use. From this virus inactivated in the presence of Triton® X-100 and $Mg^{2+}$ one aliquot was taken for plating tests (after pelleting and resuspension 10×) and safety tests in unweaned mice, and the remainder was used to prepare the experimental vaccine.

The vaccine concentration was adjusted to give 3 dosages of vaccine: 10 g per dose, using the colloidal aluminium hydroxide/saponin method (192 ml/12 ml of vaccine suspension, 1/1 dose). The dilutions were made with PBS.

Titre of control virus: $2.8 \times 10^9$ PFU/ml.

Plating control of inactivated virus: all dilutions were negative.

Safety in unweaned mouse: negative for all the animal inoculated with the inactivated virus.

9) Potency test: guinea pig 50% infective dose

$C_3$ Resende virus passed 8 times in guinea pig, processed in accordance with SELAB (SENASA) standards, was used. It was calculated that in 50% of the animals the lesion generalised in 5/7 days with $10^{-4}$ dilution of said suspension of virus.

Groups of guinea pigs were inoculated with the vaccine prepared in accordance with point 6) and according to the following design:

A) Negative control:    7 guinea pigs

B) Challenge control:    5 guinea pigs

C) 10 g dose:    8 guinea pigs

D) 5 g dose:    5 guinea pigs

E) 2.5 g dose:    5 guinea pigs

For the first 8 days the guinea pigs were observed for detection of any viral lesions produced by the vaccine, with a negative result for all of them. At 21 days after the vaccination, groups B, C, D and E were challenged with $10^{-3}$ dilution of $C_3$ Resende virus passed in guinea pig, 8th passage, and by the 7th day generalisation of the virus was observed.

In the adjoining drawings, the following is illustrated:

— The recovery of 140S virions after incubation with ammonium salts under different conditions (figure 1).

— The intactness of the genomic RNA extracted from each group of virions of figure 1 (figure 2).

— The VP1 Fragments. The partially purified virus, produced in BHK monolayer, was analysed in urea-free SDS Acrylamide (Laemmli). C=un-inactivated control virus, I=inactivated virus, same quantity as C, I TWICE=same as C but twice the quantity seeded. The virus was labeled with a mixture of radioactive AA, fluoroscopy was carried out. (figure 3).

— $A_{24}$ cruzeiro-kinetics-residual activity after inactivation (figure 4).

— $C_3$ resende-kinetics-residual activity after inactivation (figure 5).

— $C_3$ resende-residual activity after inactivation (figure 6).

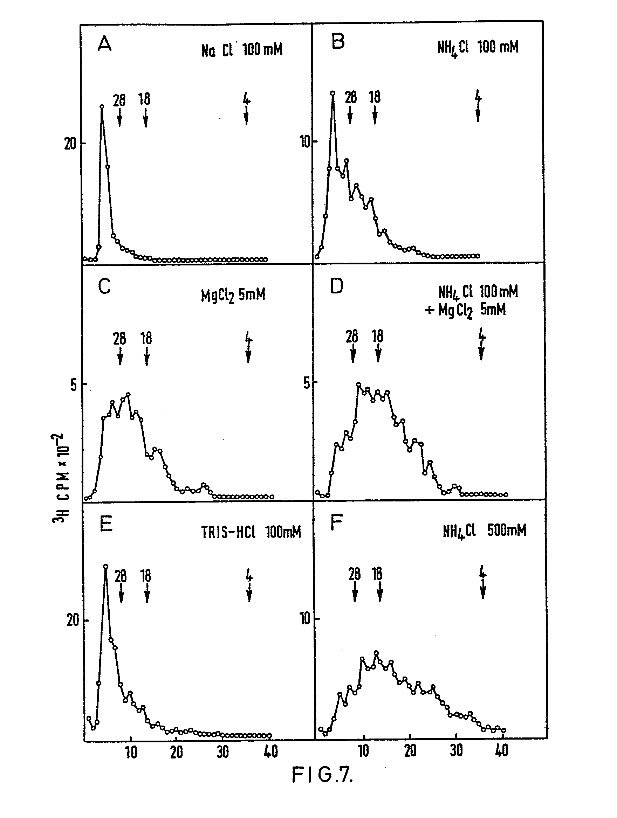

— The effect of mono and bivalent cations on the activity of the FMDV endoribonuclease (figure 7).

Eagle's Medium
Glasgow Medium=Mem Gibco
(Cat. 410—2100)

| | |
|---|---|
| Mem Gibco 10× | 100 ml |

Supplemented with:

| | |
|---|---|
| Calf Serum | 100 ml |
| Triptose Phosphate Broth 10× | 100 ml |
| Glutamine 200× | 25 ml |
| Antibiotics 1000× | 1 ml |
| $NaHCO_3$ 7.5% | 3 ml |
| $H_2O$ | 700 ml |

**Claims**

1. A process for inactivating foot-and-mouth disease virus, characterised in that it comprises incubating said virus in the presence of ammonium, potassium or cesium cations provided by a source present at a concentration of at least 100 m molar and at the temperature of 30—40°C and then recovering inactivated stable virus.

2. A process according to Claim 1, in which the incubation is effected at a pH of about 8.5.

3. A process according to Claim 1, characterised in that said incubation is effected in the presence of a non-ionic detergent.

4. A process according to Claim 3, characterised in that said non-ionic detergent is polyethyleneglycol p-isooctylphenyl ether.

5. A process according to any of Claims 1 to 4, characterised in that the culture medium also contains $Mg^{2+}$ and/or $Ca^{2+}$ ions.

6. A process according to Claim 5, in which the $Mg^{2+}$ and/or $Ca^{2+}$ ions are provided by a source present at a concentration of 2—5 m molar.

7. A process for the preparation of an inactivated foot-and-mouth disease virus vaccine, characterised in that FMDV is inactivated by a process according to any of Claims 1 to 6 without substantial loss of antigenicity, and is formulated in a vaccine carrier.

## Revendications

1. Procédé d'inactivation du virus de la fièvre aphteuse caractérisé en ce qu'il comprend: incuber ledit virus en présence de cations d'ammonium, de potassium ou de césium fournis par une source présentant une concentration d'au moins 100 mM, et une température de 30—40°C, puis récupérer le virus stable inactivé.

2. Procédé selon la revendication 1 dans lequel l'incubation est réalisée avec un pH d'environ 8,5.

3. Procédé selon la revendication 1 caractérisé en ce que ladite incubation est réalisée en présence d'un détergent non-ionique.

4. Procédé selon la revendication 3 caractérisé en que ledit détergent non-ionique est l'éther p-isooctylphénylique du polyéthyleneglycol.

5. Procédé selon l'une quelconque des revendications 1 à 4 caractérisé en ce que le milieu de culture contient également des ions $Mg^{2+}$ et/ou $Ca^{2+}$.

6. Procédé selon la revendication 5 caractérisé en ce que les ions $Mg^{2+}$ et/ou $Ca^{2+}$ sont fournis par une source présentant une concentration de 2—5 mM.

7. Procédé de préparation d'un vaccin de virus de la fièvre aphteuse inactivé, caractérisé en ce que ledit virus est inactivé au moyen d'un procédé selon l'une quelconque des revendications 1 à 6 sans perte substantielle de sa capacité antigénique, la formule vaccinale comprenant un adjuvant de l'immunité.

## Patentansprüche

1. Verfahren zum Inaktivieren von Viren bei Fuss- und Munderkrankungen, dadurch gekennzeichnet, dass man den Virus in Gegenwart von Ammonium-, Kalium- oder Cäsiumkationen, die durch eine Quelle, welche in einer Konzentration von wenigstens 100 mMolar vorliegt, zur Verfügung gestellt werden, bei einer Temperatur von 30 bis 40°C inkubiert und dann den inaktivierten stabilen Virus gewinnt.

2. Verfahren gemäss Anspruch 1, bei welchem die Inkubation bei einem pH von etwa 8,5 durchgeführt wird.

3. Verfahren gemäss Anspruch 1, dadurch gekennzeichnet, dass die Inkubation in Gegenwart eines nicht-ionischen Detergens durchgeführt wird.

4. Verfahren gemäss Anspruch 3, dadurch gekennzeichnet, dass das nichtionische Detergens Polyethylenglykol-p-isooctylphenylether ist.

5. Verfahren gemäss einem der Ansprüche 1 bis 4, dadurch gekennzeichnet, dass das Kulturmedium auch $Mg^{2+}$—und/oder $Ca^{2+}$—Ionen enthält.

6. Verfahren gemäss Anspruch 5, bei welchem die $Mg^{2+}$—und/oder $Ca^{2+}$—Ionen mittels einer Quelle, die in einer Konzentration von 2 bis 5 mMolar vorliegt, zur Verfügung gestellt werden.

7. Verfahren zur Herstellung eines inaktivierten Virusimpfstoffes gegen Fuss- und Munderkrankungen, dadurch gekennzeichnet, dass FMDV (Viren bei Fuss- und Munderkrankungen nach einem der Verfahren gemäss Ansprüchen 1 bis 6 ohne wesentlichen Verlust an Antigenizität inaktiviert wird und in einen Impfstoffträger formuliert wird.

FIG.1.

FIG.2.

C    I    I

— P—56

— Vp 0

— Vp 1—3

— Vp₁ ⎱
       ⎰ FRAG VP1
— Vp₁ ⎰

— Vp 4

FIG.3.

FIG.4.

0 063 661

FIG.5.

FIG.6.

FIG.7.